(19) Europäisches Patentamt
European Patent Office
Office européen des brevets

(11) Veröffentlichungsnummer: **0 013 742**
**B1**

# (12) EUROPÄISCHE PATENTSCHRIFT

(45) Veröffentlichungstag der Patentschrift: **22.12.82**

(21) Anmeldenummer: **79105218.6**

(22) Anmeldetag: **17.12.79**

(51) Int. Cl.³: **C 07 C 143/74**, C 07 C 149/24, C 07 C 147/12, C 07 C 149/425, C 07 C 161/02, C 07 C 149/30, C 07 C 147/14, C 07 C 143/80, C 07 C 143/86, C 07 C 147/02, C 07 C 143/825

(54) **Substituierte Sulfonsäureanilide, ihre Herstellung und Verwendung.**

(30) Priorität: **10.01.79 DE 2900685**

(43) Veröffentlichungstag der Anmeldung: **06.08.80 Patentblatt 80/16**

(45) Bekanntmachung des Hinweises auf die Patenterteilung: **22.12.82 Patentblatt 82/51**

(84) Benannte Vertragsstaaten: **AT BE CH DE FR GB IT LU NL SE**

(56) Entgegenhaltungen:
**DE-A-2 118 190**
**DE-A-2 406 475**
**DE-A-2 412 578**
**US-A-3 639 474**
**US-A-3 793 372**
**US-A-3 981 914**

(73) Patentinhaber: **BASF Aktiengesellschaft, Carl-Bosch-Strasse 38, D-6700 Ludwigshafen (DE)**

(72) Erfinder: **Stubenrauch, Gerd, Dr., Rubensstrasse 36, D-6700 Ludwigshafen (DE)**
Erfinder: **Hamprecht, Gerhard, Dr., Rote-Turm-Strasse 28, D-6940 Weinheim (DE)**
Erfinder: **Eicken, Karl, Dr., Waldstrasse 63, D-6706 Wachenheim (DE)**
Erfinder: **Jung, Johann, Dr., Hardenburgstrasse 19, D-6703 Limburgerhof (DE)**

**EP 0 013 742 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Substituierte Sulfonsäureanilide, ihre Herstellung und Verwendung

Die vorliegende Erfindung betrifft neue Sulfonsäureanilide, Verfahren zur Herstellung dieser Verbindungen, ihre Verwendung als Wachstumsregulatoren sowie Herbizide, Mittel und Mischungen, die diese Wirkstoffe enthalten, Verfahren zur Herstellung dieser Mittel und Mischungen mit deren Anwendung in der Landwirtschaft.

Es ist bekannt, dass fluorierte Alkansulfonsäureanilide als Wachstumsregulatoren und Herbizide verwendet werden können (US-PS 3 639 474), insbesondere 5-Acetamido-2-methyl-trifluormethansulfonsäureanilid und 5-Acetamido-2-chlor-trifluormethansulfonsäureanilid (US-PS 3 639 474) sowie 5-Acetamido-2,4-dimethyl-trifluormethansulfonsäureanilid (US-PS 3 894 078, US-PS 4 013 444, DE-OS 24 06 475, DE-OS 24 12 578, J. Agr. Food Chem. 22 [1974] 1111). Es ist weiterhin bekannt, dass am Sulfonamid-Stickstoff substituierte N-Carbonylfluoralkansulfonsäureanilide (DE-OS 19 17 821) Fluoralkansulfonamido-diaryl-(thio)-ether (BE-PS 765 558, DE-OS 21 18 190), N-Alkylsulfonylperfluoralkylsulfonanilide (US-PS 3 981 914) und N-Cyano-halogenalkansulfonsäureanilide (US-PS 3 793 372) als Wachstumsregulatoren und Herbizide wirksam sind. Die Wirkung und insbesondere die Selektivität dieser Stoffe ist jedoch nicht befriedigend. Schliesslich sind Benzoylalkansulfonanilide mit antiinflammatorischer, antipyretischer und analgetischer Wirkung bekannt (US-PS 3 725 451).

Gegenstand der Erfindung sind substituierte Sulfonsäureanilide der Formel I

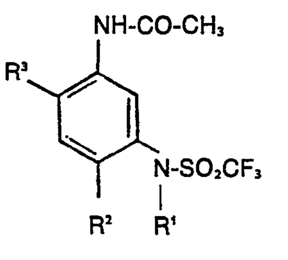

(I)

worin

$R^1$ einen gegebenenfalls durch Halogen, Cyano, Thiocyanato, Azido, Nitro, einen $C_{1-4}$-Alkoxy-, $C_{6-10}$-Aryloxy-, $C_{1-4}$-Alkylthio-, $C_{6-8}$-Arylthio-, $C_{1-4}$-Alkylsulfinyl-, $C_{1-4}$-Alkylsulfonyl-, Arylsulfinyl-, Arylsulfonyl-, $C_{1-5}$-Alkanoyl-, $C_{1-5}$-Alkoxycarbonyl-, $C_{1-5}$-Alkyl- oder -Dialkylcarbamoyl-, $C_{1-4}$-Alkanoylamino-, $C_{1-4}$-Halogenalkanoylamino-, $C_{1-4}$-Alkanoyloxy-, $C_{1-4}$-Alkanoylthio-, $C_{1-4}$-Alkoxycarbonylamino-, $C_{7-13}$-Aroyloxy-, $C_{1-6}$-Alkyl- oder -Dialkylamino-, $C_{6-12}$-Arylamino-, $C_{1-6}$-Alkyl- oder -Dialkylcarbamoylamino-, $C_{1-6}$-Alkyl- oder -Dialkylcarbamoyloxy-, $C_{1-4}$-Alkyl- oder -Dialkylaminosulfonyloxyrest oder Kombinationen dieser Gruppen substituierten Alkyl-, Alkenyl-, Alkinyl-, Cycloalkyl-, Cycloalkenyl- (mit jeweils bis zu 12 Kohlenstoffatomen) oder Arylalkylrest mit 7-14 Kohlenstoffatomen, Cyano, Thiocyanato, einen gegebenenfalls $C_{1-4}$-alkylsubstituierten Amino-, Aminosulfonyl-, Guanyl-, $N^2$-Hydroxyguanyl-, Hydroximoyl- oder $C_{1-5}$-Alkoxyimidoylrest, einen gegebenenfalls halogensubstituierten $C_{1-4}$-Alkylsulfenyl-, $C_{1-4}$-Alkylsulfinyl- oder einen gegebenenfalls halogen- und/oder $C_{1-4}$-alkylsubstituierten Arylsulfinyl- oder Arylsulfonylrest mit bis zu 10 Kohlenstoffatomen im Arylrest bedeutet,

$R^2$ und $R^3$ gleich oder verschieden sind und Halogen, Cyano, Thiocyanato, Nitro, einen gegebenenfalls halogensubstituierten $C_{1-4}$-Alkyl-, $C_{1-4}$-Alkoxy-, $C_{1-4}$-Alkylthio-, $C_{1-4}$-Alkylsulfinyl-, $C_{1-4}$-Alkylsulfonyl- oder $C_{1-4}$-Alkanoylrest bedeuten.

$R^1$ kann bedeuten: Methyl, Ethyl-, n-Propyl-, Isopropyl, Cyclopropyl, n-Butyl, Isobutyl, tert.-Butyl, Cyclobutyl, n-Pentyl, 2-Pentyl, 3-Pentyl, tert.-Amyl, Neopentyl, 2-Methyl-butyl, 3-Methyl-butyl, 3-Methyl-2-butyl, Cyclopentyl, n-Hexyl, 4-Methyl-2-pentyl, 2,3-Dimethylbutyl, 2-Methyl-2-pentyl, 2-Hexyl, 3-Hexyl, 3-Methyl-2-pentyl, 3-Methyl-3-pentyl, 4,4-Dimethylbutyl, Cyclohexyl, Heptyl, 2-Heptyl, 3-Heptyl, 4-Heptyl, Cycloheptyl, 1-Octyl, 2-Octyl, 3-Octyl, 4-Octyl, 6-Octyl, 5-Ethyl-2-heptyl, 2,6-Dimethyl-4-heptyl, 7-Ethyl-2-methyl-4-nonyl, 2,4-Dimethyl-3-pentyl, 3-Methyl-2-heptyl, 5-Ethyl-2-nonyl, Nonyl, Decyl, Undecyl, Dodecyl, 6-Ethyl-3-decyl, 6-Ethyl-3-octyl, 2-Methyl-2-pentyl, 2,3-Dimethyl-2-butyl, 2-Methyl-2-hexyl, 3-Ethyl-3-pentyl, 3-Methyl-3-hexyl, 2,3-Dimethyl-pentyl-3, 2,4-Dimethyl-2-pentyl, 2,2,3-Trimethyl-3-butyl, 2-Methyl-2-heptyl, 4-Methyl-4-heptyl, 2,4-Dimethyl-2-hexyl, 2-Methyl-2-octyl, 1-Methyl-1-cyclopentyl, 1-Methyl-1-cyclohexyl, 1-Ethyl-1-cyclohexyl, Chlor-tert.butyl, 1,1-Dichlor-2-methyl-2-propyl, 1,3-Dichlor-2-methyl-2-propyl, 1-Cyclohexyl-1-ethyl, 1-Chlorethyl, 2-Chlorethyl, 1-Chlorpropyl, 2-Chlorpropyl, 3-Chlorpropyl, 1-Chlor-2-propyl, 2-Chlorbutyl, 2-Chlor-2-methyl-3-propyl, 1-Fluorethyl, 2-Fluorethyl, 2-Fluorpropyl, 3-Fluorpropyl, 1-Fluor-2-propyl, 2-Fluorbutyl, 2-Fluor-2-methyl-3-propyl, 2-Bromethyl, 3-Brompropyl, 4-Chlorbutyl, 2-Chlorcyclohexyl, 1,1,1-Trifluorisopropyl, Hexafluor-2-methyl-isopropyl, Hexafluorisopropyl, Hexachlorisopropyl, 1,2-Dibrom-allyl, 2,2,2-Trifluorethyl, 1-Chlorbutin-2-yl-4, 3-Chlor-butin-1-yl-4, 1-Chlor-buten-2-yl-4, 2,3-Dibrom-1-propyl, 2,2,2-Trichlorethyl, 1-Chlorpentin-2-yl-4, 2,2,2-Tribromethyl, 3,4,4-Trichlorbuten-3-yl-2, 1-Brom-2-propyl, 1,3-Dibrom-2-propyl, 3-Chlorbuten-1-yl-4, Allyl, Methallyl, Crotyl, 2-Ethyl-hexen-2-yl-1, Hexen-5-yl-1, Undecen-10-yl-1, 2-Methyl-buten-2-yl-1, 2-Methylbuten-1-yl-3, Butin-1-yl-3, Butin-2-yl-1, Buten-1-yl-3, Propargyl, 2-Methylbuten-1-yl-4, 2-Methyl-buten-2-yl-4, 3-Methylbuten-1-yl-3, 1-Ethinylcyclohexyl, Methoxyethyl, Ethoxyethyl, 3-Methoxypropyl, Methoxyisopropyl, 3-Methoxybutyl, 1-Methoxybutyl-2, Ethoxy-tert.butyl, Methoxy-tert.butyl, Cyclohexoxy-tert.butyl, 2-Methoxy-butyl, 4-Methoxy-butyl, Methyl-mercapto-ethyl, Ethylmercaptoethyl, 3-Methylmercapto-propyl, 3-Methyl-mercaptobutyl, 1-Methylmercapto-butyl-2, Methylmercapto-tert.-

butyl, 2-Methylmercaptobutyl, 4-Methylmercaptobutyl, 3-n-Butoxyethyl, 2-Ethoxypropyl, 3-Ethoxy-2-propyl, 2-Methylbutanon-3-yl-1, 2-Methylpentanon-4-yl-2, 3-Butanon-1-yl, 3-Butanon-2-yl, 2-Propanon-1-yl, 2-Pentanon-1-yl, Methylacetat-2, Ethylacetat-2, Methylpropionat-2, Methylpropionat-3, 2-Ethoxy-ethylacetat-2, 2-Methoxy-methylpropionat-2, Methyl-(2-vinyl-propionat-2), Methylcarbamoyl-methyl, Dimethylcarbamoyl-methyl, Cyano, Thiocyanato, Guanyl, $N^2$-Methylguanyl, $N^1$-Isopropylguanyl, $N^1,N^1$-Dimethylguanyl, $N^1,N^1,N^2$-Trimethylguanyl, $N^2$-Hydroxyguanyl, $N^2$-Methoxyguanyl, Methoxy-imidoyl, Isopropoxyimidoyl, Methancarbohydroximoyl, Benzohydroximoyl, O-Methyl-benzo-hydroximoyl, Aminocarbonylmethyl, Aminocarbonyl-ethyl, Amino, Dimethylamino, N-Isopropyl-N-methylamino, Methylamino, Isopropylamino, Ethylsulfenyl, 2,4-Dinitrophenylsulfenyl, Dichlorfluor-methylsulfenyl, Trichlormethyl-sulfenyl, N-Methylsulfamoyl, N-Isopropyl-N-methylsulfamoyl, α-Cyanoethyl, β-Cyanoethyl, Cyanomethyl, Rhodanomethyl, Methylsulfinyl-methyl, Benzysulfinyl-methyl, 4-Methyl-sulfinyl-butyl, 1-Methylsulfonyl-butyl-2, Ethylsulfonylethyl, Methylsulfonyl-methyl, p-Chlorbenzylsulfonyl-methyl, Phenylsulfonyl-methyl, Azidomethyl, β-Azidoethyl, Acetylamino-methyl, p-Chlorbenzoylamino-methyl, Propionyloxy-methyl, Acetoxymethyl, N-(2,6-Dimethylphenyl)-N-methoxycarbonyl-aminomethyl, N-(2-Ethylphenyl)-N-ethoxy-carbonyl-aminomethyl, N-(2-Methyl-6-ethyl-phenyl)-N-chloracetyl-aminomethyl, β-N,N-Dimethylamino-ethyl, β-N,N-Diisopropylamino-ethyl, β-N-Phenylamino-ethyl, Piperidinomethyl, Dimethylamino-methyl, Morpholinomethyl, Methylsulfonyloxymethyl, N-Methylaminosulfonyloxy-methyl.

Eine besonders bevorzugte Klasse von Sulfonsäureaniliden der allgemeinen Formel (I) ist dadurch gekennzeichnet, dass $R^1$ einen gegebenenfalls durch Halogen, Cyano, Thiocyanato oder Azido in den Positionen α oder β zum Sulfonamid-Stickstoff substituierten $C_{1-4}$-Alkylrest, eine $C_{1-3}$-Alkylgruppe, die am α- oder β-Kohlenstoffatom durch einen $C_{1-4}$-Alkoxy, $C_{1-3}$-Alkylsulfinyl-, $C_{1-2}$-Alkylsulfonyl-, N-Chloracetyl-N-arylamino-, $C_{1-4}$-Dialkyl-carbamoylamino-, $C_{1-4}$-Alkoxycarbamoyl-, $C_{1-6}$-Alkanoyloxy- oder $C_{7-13}$-Aroyloxyrest substituiert sein kann, einen gegebenenfalls halogensubstituierten Alkenyl- oder Alkinylrest mit 3 bis 4 Kohlenstoffatomen, Cyano oder Thiocyanato bedeutet, und $R^2$ und $R^3$ unabhängig voneinander für Methyl, Halogen, Methoxy, Methylthio oder Methylsulfonyl stehen.

Die Bezeichnung «Halogen» bedeutet vorzugsweise Fluor, Chlor und Brom; «Cycloalkyl» bedeutet vorzugsweise Cyclopropyl, Cyclobutyl, Cyclopentyl, Cyclohexyl und Cycloheptyl.

Gegenstand der Erfindung ist weiter ein Verfahren zur Herstellung der substituierten Sulfonsäureanilide der allgemeinen Formel (I), welches darin besteht, dass man

a) Verbindungen der Formel (II)

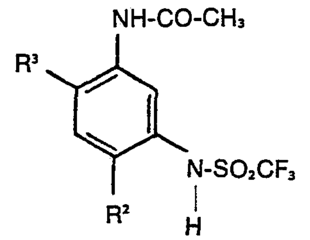

(II)

worin $R^2$ und $R^3$ die oben genannten Bedeutungen besitzen, mit einer Verbindung der Formel (III)

$R^1$-L (III)

worin $R^1$ die oben genannten Bedeutungen besitzt und L eine nucleophil verdrängbare Austrittsgruppe, insbesondere Halogen, Hydrogensulfat, Halogensulfonat, einen gegebenenfalls substituierten Alkylsulfonyloxy-, Arylsulfonyloxy-, Alkylsulfat-, Oxonium-, Sulfonium- oder Ammoniumrest bedeutet, gegebenenfalls in Gegenwart eines Verdünnungsmittels und/oder eines Säurefängers und/oder eines Reaktionsbeschleunigers bei Temperaturen zwischen 0 und 170°C umsetzt, oder

b) Verbindungen der Formel (IV)

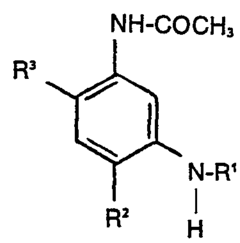

(IV)

worin $R^1$, $R^2$ und $R^3$ die oben genannten Bedeutungen haben, mit einem Sulfonierungsmittel der Formel (V)

$CF_3$-$SO_2$-Z (V)

worin Z eine Austrittsgruppe, insbesondere Halogen oder den Anhydridrest $CF_3$-$SO_2O$- bedeutet, gegebenenfalls in Gegenwart eines Verdünnungsmittels und gegebenenfalls in Gegenwart einer organischen oder anorganischen Base, bei Temperaturen zwischen −60 und +120°C umsetzt.

Anstelle der freien Wasserstoffverbindungen der Formeln (II) bzw. (IV) können auch entsprechende Salze, die nach bekannten Methoden in einer Vorreaktion oder gegebenenfalls durch Zugabe der äquivalenten Menge einer organischen oder anorganischen Base in situ erzeugt werden, beispielsweise Alkali-, Erdalkali- oder gegebenenfalls alkylsubstituierte Ammoniumsalze, eingesetzt werden.

Ein auf diese Weise erhaltenes Derivat mit $R^1$ = Alkylthioalkyl bzw. Arylthioalkyl kann gegebenenfalls zur entsprechenden Sulfinyl- bzw. Sulfonylverbindung oxidiert werden.

Die Derivate, in denen $R^1$ eine Cyanogruppe ist, können mit Aminen, Alkoholen oder Hydroxylaminen zu den entsprechenden Guanyl- bzw. Alkoxyimidoylverbindungen umgesetzt werden.

Verwendet man 5-Acetamido-2,4-dimethyl-trifluormethan-sulfonsäureanilid und Methylbromid als Ausgangsmaterialien, kann der Reaktionsablauf der erfindungsgemässen Verfahrensvariante a) durch das folgende Schema beschrieben werden:

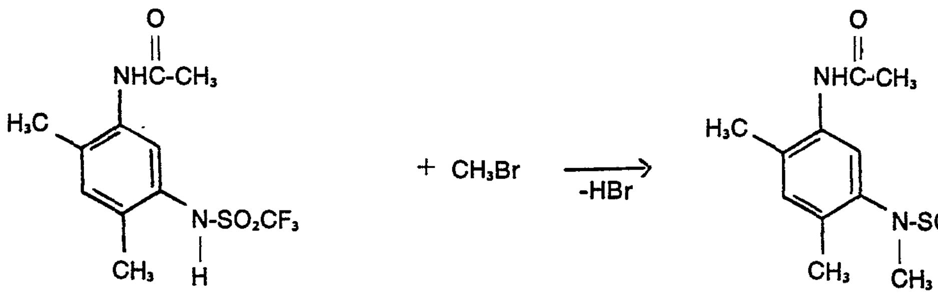


Verwendet man 5-Acetamido-2,4-dimethyl-trifluormethan-sulfonsäureanilid-Natriumsalz und Chloracetonitril als Ausgangsmaterialien und Natriumjodid als Reaktionsbeschleuniger, kann der Reaktionsablauf des erfindungsgemässen Verfahrens a) durch folgendes Schema wiedergegeben werden:

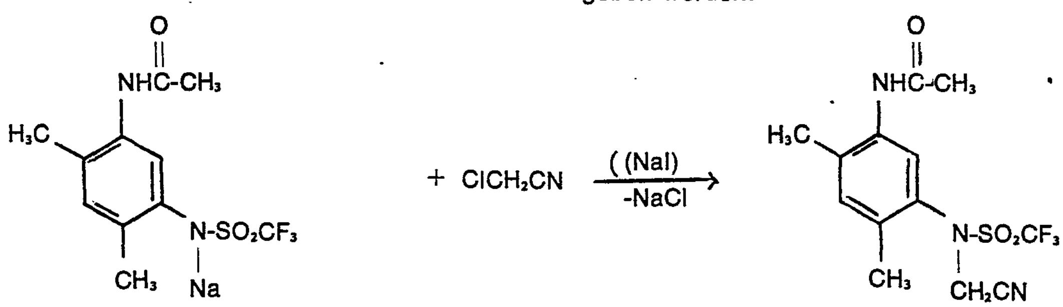


Verwendet man 5-Acetamido-2-methyl-phenyl-cyanamid und Trifluormethansulfonsäurechlorid als Ausgangsmaterialien, kann der Reaktionsablauf der erfindungsgemässen Verfahrensvariante b) durch folgendes Formelschema beschrieben werden:

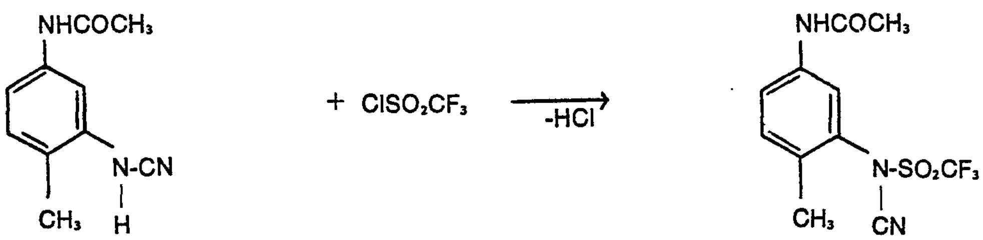


Für beide Verfahrensvarianten können beispielsweise folgende Lösungs- bzw. Verdünnungsmittel verwendet werden: Wasser; Formamide, wie Dimethylformamid, Formamid, Dimethylacetamid; Nitrile, wie Acetonitril, Benzonitril, Butyronitril; Sulfoxide, wie Dimethylsulfoxid; Phosphorsäureamide, wie Hexamethylphosphorsäuretriamid; Ketone, wie Aceton, Ethylmethylketon, Cyclohexanon, Acetophenon; Ether, wie Tetrahydrofuran, Anisol, Dimethoxyethan, n-Butylethylether, Dioxan; Nitroalkane, wie Nitromethan; Nitrobenzol; Alkohole, wie Methanol, Ethanol, Isopropanol, n-Butanol, 3-Methylbutanol; Harnstoffe, wie Tetramethylharnstoff; Etheralkohole, wie Ethylenglykolmonomethylether; Sulfone, wie Sulfolan; Ester, wie Essigsäureethylester, Propionsäuremethylester, Ameisensäuremethylester; Halogenkohlenwasserstoffe, insbesondere Chlorkohlenwasserstoffe, beispielsweise Methylenchlorid, Chloroform, 1,2-Dichlorethan, 1,1,2,2- oder 1,1,1,2-Tetrachlorethan, Dichlorpropan, Trichlorethylen, Chlorbenzol, o,m,p-Dichlorbenzol, Fluorbenzol, o,m,p-Chlortoluol, Dichlornaphthalin, Tetrachlorkohlenstoff; aliphatische oder cycloaliphatische Kohlenwasserstoffe, wie Heptan, Pinan, Benzinfraktionen innerhalb eines Siedepunktsintervalles von 70 bis 190°C, Cyclohexan, Methylcyclohexan, Dekalin, Petrolether, Ligroin, 2,2,4-Trimethylpentan, Octan; aromatische Kohlenwasserstoffe, wie Benzol, Toluol, o,m,p-Xylol, o,m,p-Cymol, Tetralin; und entsprechende Gemische. Zweckmässig verwendet man das Lösungsmittel in einer Menge von 100 bis 2000 Gew.-%, vorzugsweise 100 bis 1000 Gew.-%, bezogen auf die Ausgangsverbindungen (II) bzw. (IV).

Die erfindungsgemässen Verfahren a) und b) werden vorzugsweise in Gegenwart einer organischen oder anorganischen Base vorgenommen. Insbesondere werden basische Hydroxyverbindungen, basische Oxide, tertiäre Amine, Alkoholate, Carbonate oder Hydride verwendet, beispielsweise Kaliumhydroxid, Natriumhydroxid, Kaliumcarbonat, Calciumoxid, Bariumoxid, Magnesiumhydroxid, Magnesiumoxid, Bariumhydroxid, Calciumcarbonat, Magnesiumcarbonat, Magnesiumbicarbonat, Zinkhydroxid, Zinkoxid, Zinkcarbonat, Zinkbicarbonat, Natriummethylat, Magnesiumethylat, Kaliumethylat, Natriumpropylat, Aluminiumisopropylat, Natriumbutylat, Lithiummethylat, Calciumcyclohexanolat, Natriumisopropylat, Kalium-tert.butylat, Trimethylamin, Triethylamin, Tripropylamin, Triisopropylamin, Tributylamin, Triisobutylamin, Tri-sec.butylamin, Tri-tert.butylamin, Tribenzylamin, Tricyclohexylamin, Triamylamin, Trihexylamin, N,N-Dimethylanilin, N,N-Diethylanilin, N,N-Diisopropylanilin, N,N-Dimethyltoluidin, N,N-diethyltoluidin, N,N--Dipropyltoluidin, N,N-Dimethyl-p-aminopyridin, N,N-Diethyl-p-aminopyridin, N-Methylpiperidin, N-Ethylpiperidin, N-Methylpyrrolidin, N-Ethylpyrrolidin, N-Methylpyrrol, N-Methylmorpholin, N--Ethylmorpholin, N-Methylhexamethylenimin, Pyridin, Chinolin, α,β,γ-Picolin, N,N,N',N'-Tetramethylethylendiamin, N-Ethyldiisopropylamin, N,N--Dimethylcyclohexylamin, Natriumhydrid, Lithiumhydrid, Calciumhydrid eingesetzt. Es können aber auch andere, üblicherweise verwendete basische Stoffe verwendet werden. Als Reaktionsbeschleuniger kommen vorzugsweise Metall-Iodide und -Bromide, beispielsweise Natriumiodid, Kaliumbromid, Calciumiodid; Kronenether; quartäre Ammoniumverbindungen, wie Tetrabutylammoniumiodid oder -bromid; oder Kombinationen dieser Stoffe in Frage.

Das Verfahren a) wird im allgemeinen bei 0 bis 170°C, vorzugsweise bei 0 bis 140°C, während 30 Minuten und 100 Stunden, vorzugsweise 1 Stunde und 24 Stunden, drucklos oder unter Druck, kontinuierlich oder diskontinuierlich durchgeführt.

Im allgemeinen verwendet man beim Verfahren a) auf 1 Mol der Verbindung (II) bzw. des entsprechenden Salzes 0,5 bis 2 Mol, vorzugsweise 0,9 bis 1,5 Mol der Verbindung (III), gegebenenfalls 0,5 bis 2 Mol der Base sowie 0,01 bis 0,1 Mol des Reaktionsbeschleunigers. Zur Isolierung der Endprodukte der Formel (I) bringt man im Falle der Verwendung von mit Wasser mischbaren Lösungs- bzw. Verdünnungsmitteln die Reaktionslösung oder den nach der Entfernung des Lösungs- bzw. Verdünngsmittels verbleibenden Rückstand in verdünnte wässrige Alkalilösung ein. Scheidet sich ein Öl ab, wird es extrahiert oder die überstehende Lösung dekantiert; ein gegebenenfalls entstehender Niederschlag wird abgesaugt. Bei mit Wasser nicht mischbaren Verdünnungsmitteln extrahiert man im allgemeinen die organische Phase mit verdünnter, wässriger Alkalilösung, trocknet und engt ein. Fällt das gewünschte Produkt während der Reaktion direkt aus, kann es durch einfaches Absaugen isoliert werden. Gegebenenfalls können die Endstoffe in üblicher Weise, beispielsweise durch Umkristallisation oder Chromatogaphie, weiter gereinigt werden.

Die Verfahrensvariante b) wird im allgemeinen bei −60 bis +120°C, vorzugsweise bei −40 bis +80°C in 1 bis 100 Stunden, drucklos oder unter Druck, kontinuierlich oder diskontinuierlich durchgeführt. Im allgemeinen verwendet man auf 1 Mol der Verbindung der Formel (IV) 0,8 bis 1,3 Mol, vorzugsweise 0,9 bis 1,1 Mol, des Sulfonierungsmittels sowie 0,8 bis 2 Mol der Base. Die Isolierung des Endstoffes der Formel (I) erfolgt wie bei der Verfahrensvariante a) beschrieben.

Beispiel 1

Zu einer Aufschlämmung von 19,2 Teilen Brom in 80 Teilen Wasser tropft man unter Rühren in ca. 10 Minuten bei 0 bis 10°C eine Lösung von 5,9 Teilen Natriumcyanid in 40 Teilen Wasser. Nach Entfärbung der Mischung lässt man eine Lösung von 4 Teilen Natriumhydroxid und 31 Teilen 5-Acetamido-2,4-dimethyl-trifluormethansulfonsäureanilid in 50 Teilen Wasser zulaufen und rührt 5 Stunden bei 50°C nach. Nach dem Abkühlen alkalisiert man den Ansatz mit etwas wässriger Alkalilauge und saugt den Niederschlag ab. Nach dem Trocknen erhält man 29,5 Teile (88% der Theorie) 5-Acetamido-N'-cyano-2,4-dimethyl-trifluormethansulfonsäureanilid vom Schmelzpunkt 110 bis 111°C.

Beispiel 2

Zu einer Lösung von 49,8 Teilen 5-Acetamido--2,4-dimethyl-trifluormethylsulfonsäureanilid Na--Salz in 150 Teilen Dimethylformamid gibt man auf einmal 15,5 Teile Chlormethylmethylsulfid. Man rührt ca. 8 Stunden bei Raumtemperatur nach, giesst dann die Reaktionsmischung in Wasser, extrahiert mit Ether und wäscht die organische Phase mit Wasser und 1 N wässriger Natronlauge. Nach dem Trocknen, Einengen und Verrühren mit Diisopropylether erhält man 48,1 Teile (86,7% der Theorie) 5-Acetamido-2,4-dimethyl-N'-methylthiomethyltrifluormethansulfonsäureanilid vom Schmelzpunkt 106 bis 107°C.

Beispiel 3

Zu 17,3 Teilen 5-Acetamido-2,4-dimethyl-N--methylanilin in 100 Teilen Chloroform tropft man bei 0°C getrennt und gleichzeitig 17 Teile Trifluormethansulfonsäurechlorid und 10,3 Teile Triethylamin. Man rührt über Nacht bei Raumtemperatur nach, saugt vom Niederschlag ab, wäscht gründlich mit Chloroform nach und extrahiert die organische Phase mit Wasser und 2 N wässriger Salzsäure. Nach dem Trocknen und Einengen erhält man 24 Teile (82% der Theorie) 5-Acetamido-2,4-dimethyl-N'-methyl-trifluormethansulfonsäureanilid vom Schmelzpunkt 128-130°C.

Beispiel 4

Zu einer Suspension von 10 Teilen O-Methylhydroxylamin-hydrochlorid in 100 Teilen Ethanol gibt man 7,2 Teile Natriumcarbonat und rührt die Mischung 1 Stunde bei Raumtemperatur nach. Nach der Zugabe von 37 Teilen 5-Acetamido-N'-cyano-2,4-dimethyl-trifluormethansulfonsäureanilid rührt man 10 Stunden bei Raumtemperatur und 5 Stunden bei 50°C nach, zieht das Lösungsmittel im Vakuum ab und giesst den Rückstand in Wasser. Man extrahiert das sich abscheidende Öl mit Essigester, wäscht die organische Phase zweimal mit Wasser, trocknet und engt ein. Nach der Behandlung des Rückstandes mit Diisopropylether/Toluol erhält man 25 Teile 5-Acetamido-2,4-dimethyl-N'-(N²'-methoxy)-guanyl-trifluormethansulfonsäureanilid vom Schmelzpunkt 105 bis 107°C.

Beispiel 5

Zu 18,6 Teilen 5-Acetamido-2,4-dimethyl-N'-methylthiomethyl-trifluormethansulfonsäureanilid (Beispiel 2) in 200 Teilen Chloroform gibt man bei −10°C eine Lösung von 10 Teilen 85%iger m-Chlorperbenzoesäure in 120 Teilen Chloroform. Nach dem Stehen über Nacht wird in überschüssige, gesättigte Bicarbonatlösung in Wasser eingegossen, die organische Phase nochmals mit Bicarbonatlösung gewaschen, getrocknet und eingeengt. Nach dem Ausrühren mit Diisopropylether erhält man 16,5 Teile (82% der Theorie) 5-Acetamido-2,4-dimethyl-N'-methylsulfonylmethyl-trifluormethansulfonsäureanilid vom Schmelzpunkt 161 bis 163°C.

Nach den oben beschriebenen Verfahren wurden weitere Verbindungen der allgemeinen Formel (I) hergestellt

| Beispiel Nr. | R¹ | R² | R³ | Schmelzpunkt [°C] |
|---|---|---|---|---|
| 6 | $-CH_2CN$ | $CH_3$ | $CH_3$ | 149-151 |
| 7 | $-CH_2CN$ | F | F | |
| 8 | $-CH_2OCH_3$ | $CH_3$ | $CH_3$ | 127-129 |
| 9 | $-CH_2OC_2H_5$ | Cl | Cl | |
| 10 | $-CH_2OCH_2C_6H_5$ | Cl | $CH_3$ | |
| 11 | $-CH_2OCH_3$ | Br | Br | |
| 12 | $-CH(CO_2C_2H_5)-OCH_2H_5$ | $CH_3$ | $CH_3$ | 93-95 |
| 13 | $-CH(CH_3)-O-CH_3$ | $CH_3$ | $CH_3$ | |
| 14 | $-SCCl_3$ | $CH_3$ | $CH_3$ | |
| 15 | -SCN | $CH_3$ | $CH_3$ | |
| 16 | -SCN | Cl | Cl | |
| 17 | $-CH_2S-$ 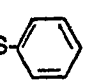 | $CH_3$ | $CH_3$ | |
| 18 | $-CH_2SCH_3$ | Cl | Cl | |
| 19 | $-CH_2S-CH_2-$ 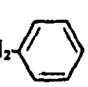 | $CH_3$ | $CH_3$ | NMR (DMSO): $CH_3$ δ 2,0; 2,05; 2,25; $CH_2$ 3,6; 4,7; arom. H 6,95-7,8 NH 8,2 |
| 20 | $-CH-S-CH_2-$ 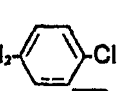 -Cl | $CH_3$ | $CH_3$ | |
| 21 | $-CH_2-SO_2-CH_2-$ 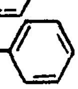 | $CH_3$ | $CH_3$ | |
| 22 | CN | Cl | Cl | |
| 23 | CN | Br | Br | |

| Beispiel Nr. | $R^1$ | $R^2$ | $R^3$ | Schmelzpunkt [°C] |
|---|---|---|---|---|
| 24 | CN | $CH_3$ | $CH_3CO$ | |
| 25 | CN | $CH_3$ | $CH_3O$ | |
| 26 | CN | $CH_3$ | $CH_3S$- | |
| 27 | CN | $CH_3O$ | $CH_3$ | |
| 28 | CN | $CH_3$ | $CH_3SO_2$- | |
| 29 | $CH_2SCN$ | $CH_3$ | $CH_3$ | |
| 30 | $CH_2SCN$ | $CH_3O$- | Cl | |
| 31 | i-$C_3H_7$ | $CH_3$ | $CH_3$ | |
| 32 | $C_2H_5$ | $CH_3O$- | H | |
| 33 | $-CH_2-CH=CH_2$ | $CH_3$ | $CH_3$ | 125-127 |
| 34 | 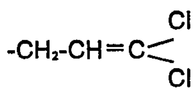 | $CH_3$ | $CH_3$ | |
| 35 | 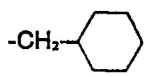 | $CH_3$ | $CH_3$ | |
| 36 | $-CH_2-C=C-CH_2$ | $CH_3$ | $CH_3$ | |
| 37 | $-CH_2C\equiv CH$ | $CH_3$ | $CH_3$ | 158-160 |
| 38 | $-CH_2CH_2Cl$ | $CH_3$ | $CH_3$ | |
| 39 | 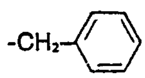 | $CH_3$ | $CH_3$ | |
| 40 | 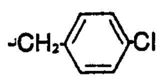 | $CH_3$ | $CH_3$ | 122-124 |
| 41 | 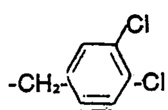 | $CH_3$ | $CH_3$ | |
| 42 | 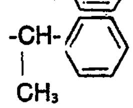 | $CH_3$ | $CH_3$ | |
| 43 | $-N(CH_3)_2$ | $CH_3$ | $CH_3$ | |
| 44 | i-$C_3H_7NH$- | $CH_3$ | $CH_3$ | |
| 45 | $(CH_3)_2NSO_2$- | $CH_3$ | $CH_3$ | |
| 46 | i-$C_3H_7NHSO_2$- | $CH_3$ | $CH_3$ | |
| 47 | sec-$C_4H_9NHSO_2$- | $CH_3$ | $CH_3$ | |
| 48 | 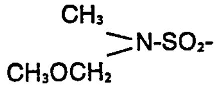 | $CH_3$ | $CH_3$ | |
| 49 | 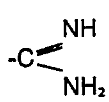 | $CH_3$ | $CH_3$ | |
| 50 | 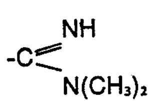 | $CH_3$ | $CH_3$ | |

| Beispiel Nr. | R¹ | R² | R³ | Schmelzpunkt [°C] |
|---|---|---|---|---|
| 51 | 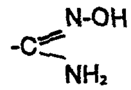 | $CH_3$ | $CH_3$ | |
| 52 | 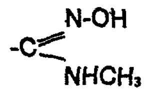 | $CH_3$ | $CH_3$ | |
| 53 | 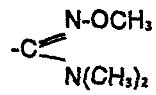 | $CH_3$ | $CH_3$ | |
| 54 | 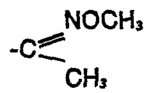 | $CH_3$ | $CH_3$ | |
| 55 | 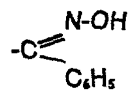 | $CH_3$ | $CH_3$ | |
| 56 | 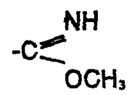 | $CH_3$ | $CH_3$ | |
| 57 | 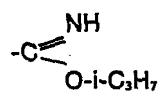 | $CH_3$ | $CH_3$ | |
| 58 | $-CH_2COCH_3$ | $CH_3$ | $CH_3$ | 52-55 |
| 59 | 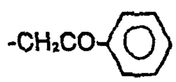 | $CH_3$ | $CH_3$ | |
| 60 | $-CH_2CO_2CH_3$ | $CH_3$ | $CH_3$ | |
| 61 | 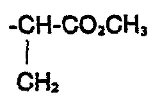 | $CH_3$ | $CH_3$ | 109-111 |
| 62 | $-CH_2-CONH_2$ | $CH_3$ | $CH_3$ | |
| 63 | 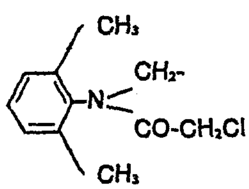 | $CH_3$ | $CH_3$ | 204 |
| 64 | 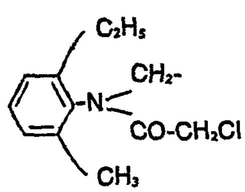 | $CH_3$ | $CH_3$ | 206 |
| 65 | 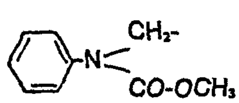 | $CH_3$ | $CH_3$ | |
| 66 | $-CH_2OCOCH_3$ | $CH_3$ | $CH_3$ | 130-133 |

| Beispiel Nr. | $R^1$ | $R^2$ | $R^3$ | Schmelzpunkt [°C] |
|---|---|---|---|---|
| 67 | $-CH_2OCO-CH{=}CH_2$ | $CH_3$ | $CH_3$ | |
| 68 | 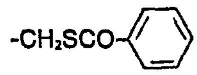 | $CH_3$ | $CH_3$ | |
| 69 | 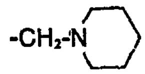 | $CH_3$ | $CH_2$ | |
| 70 | 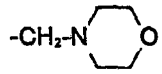 | $CH_3$ | $CH_3$ | |
| 71 | $-CH_2NHCO-N(CH_3)_2$ | $CH_3$ | $CH_3$ | |
| 72 | $-CH_2NH\overset{S}{\overset{\Vert}{C}}-N(CH_3)_2$ | $CH_3$ | $CH_3$ | |
| 73 | $-CH_2OCO-NHCH_3$ | $CH_3$ | $CH_3$ | |
| 74 | $-CH_2OSO_2-NHCH_3$ | $CH_3$ | $CH_3$ | |
| 75 | $-CH-O-\overset{O}{\overset{\Vert}{C}}-OC_2H_5$ | $CH_3$ | $CH_3$ | 143-145 |
| 76 | 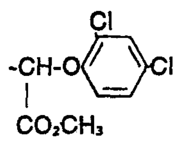 | $CH_3$ | $CH_3$ | 164-165 |
| 77 | 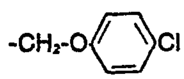 | $CH_3$ | $CH_3$ | 96-99 |

Die neuen Wirkstoffe haben eine starke biologische Wirkung auf Pflanzen, d.h. sie beeinflussen das Pflanzenwachstum, sei es als Hemmung des Längenwachstums, sei es als Veränderung der Konzentration der Pflanzeninhaltsstoffe, sei es als Vernichtung unerwünschter Pflanzen bei gleichzeitiger Schonung der Nutzpflanzen.

Die Anwendung erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Napthaline oder deren Derivate z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon und Wasser in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Napththalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Staubmittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kalk, Kreide, Talkum, Bolus, Löss, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen erhalten zwischen 0,1 und 95 Gew.-% Wirkstoff, vorzugsweise zwischen 0,5 und 90 Gew.-%.

In verschiedenen Fällen kann sich die Kombination bzw. Mischung der erfindungsgemässen Substanzen mit anderen wachstumsregulierenden Wirkstoffen als vorteilhaft erweisen, wie u. a. mit ethylenbildenden Stoffen verschiedener chemischer Struktur (z. B. Phosphonsäurederivate und Silane, Ethylhydrazine), Oniumverbindungen (z. B. Trimethylammonium-, Hydrazonium- und Sulfoniumsalze, Derivate von Morpholinium-, Piperidinium- und Pyridaziniumverbindungen). Von Interesse sind auch weitere wachstumsregulierende Substanzen, u. a. aus der Gruppe der Maleinsäurehydrazide, Abscissinsäurederivate, chlorierten Phenoxyfettsäuren mit auxinähnlicher Wirkung, sowie höherwertigen Alkohole und Fettsäureester mit spezifischer Wirkung auf meristematische Gewebepartien.

Die erfindungsgemässen Mittel können in ihrer aufgewandten Menge schwanken. Die aufgewandte Menge hängt hauptsächlich von der Art des gewünschten Effektes ab.

Die Aufwandmenge liegt im allgemeinen zwischen 0,1 und 15 oder mehr, vorzugsweise 0,2 bis 6 kg Wirkstoff pro Hektar.

Sie beeinflussen das Wachstum ober- und unterirdischer Pflanzenteile in verschiedener Weise und besitzen in den üblichen Anwendungskonzentrationen eine geringe Warmblütertoxizität.

Die neuen Mittel greifen in die physiologischen Vorgänge der Pflanzenentwicklung ein und können für verschiedene Zwecke verwendet werden. Die verschiedenartigen Wirkungen dieser Wirkstoffe hängen im wesentlichen ab von dem Zeitpunkt der Anwendung, bezogen auf das Entwicklungsstadium des Samens oder der Pflanze, sowie von den angewendeten Konzentrationen.

Mit den neuen Mitteln wird das vegetative und generative Pflanzenwachstum, sowie bei entsprechender Konzentration auch die Keimfähigkeit beeinflusst.

Die Beeinflussung der vegetativen Entwicklung besteht insbesondere in einer Reduzierung der Wuchshöhe, wodurch bei zahlreichen Pflanzen, insbesondere Getreide, eine erhöhte Standfestigkeit und verringerte Tendenz zu sogenannten «Lagern» bewirkt wird. Gleichzeitig wird die Bestockung verbessert, was zu einer höheren Zahl ährentragender Halme je Flächeneinheit führt.

Bei Gras wirkt sich die reduzierte Wuchshöhe in einer dichteren, resistenteren Narbe und vor allem in der Einsparung mehrerer Schnitte aus. Letzteres ist für Zierrasen, aber auch für Grasflächen entlang der Strassen und in Parkanlagen von grossem arbeitswirtschaftlichen Vorteil. Hinzu kommt, dass parallel zur Wuchshöhenreduzierung eine Zunahme des Chlorophyllgehaltes stattfindet, wodurch behandelte Grasflächen, aber auch sonstige Pflanzenbestände, eine deutlich dunklere grüne Färbung annehmen.

Die Beeinflussung des vegetativen Wuchstums bewirkt bei zahlreichen Pflanzen, wie z. B. Baumwolle und Soja, eine starke Zunahme des Blüten- und Fruchtansatzes.

Besonders hervorzuheben ist, dass das Wurzelwachstum durch die Behandlung mit den erfindungsgemässen Substanzen gefördert wird. Dies führt zu einer rationelleren Wasser- und Nährstoffausnutzung durch die behandelten Pflanzen; dabei wird nicht nur die Trockenheitssondern auch die Kälteresistenz (Frostresistenz) erhöht.

Vielseitig und umfangreich sind auch die Anwendungsmöglichkeiten im Obst- und Zierpflanzenbau, sowie im Landschaftsbau einschliesslich der sachgemässen Vegetationsbeeinflussung auf Brachflächen, sowie auf Flug- und Übungsplätzen.

Bei der Beeinflussung von Blüh- und Reifevorgängen, sowie im Rahmen spezieller Anzuchtverfahren, lassen sich die Substanzen ebenfalls erfolgreich anwenden.

Nicht zuletzt können die neuen Mittel auch die Konzentration wichtiger Pflanzeninhaltsstoffe wie Zucker und Proteine positiv beeinflussen.

Das Ausmass und die Art der Wirkung sind von verschiedenen Faktoren abhängig, insbesondere von Applikationszeit in Bezug auf das Entwicklungsstadium der Pflanze und der Anwendungskonzentration. Diese Faktoren sind aber wiederum je nach Pflanzenart und dem

gewünschten Effekt verschieden. So wird man beispielsweise Rasenflächen während der gesamten Wachstumsperiode behandeln; Zierpflanzen, bei denen die Intensität und Anzahl der Blüten erhöht werden soll, vor Ausbildung der Blütenanlage; Pflanzen, deren Früchte verwendet resp. verwertet werden, in entsprechendem Abstand vor der Ernte. Verschiedene Derivate der hier beschriebenen Verbindungsklasse haben herbizide Eigenschaften. Sie eignen sich daher zur Beseitigung und Niederhaltung von unerwünschtem Pflanzenwuchs.

Beispiele für Formulierungen sind:

I 20 Gewichtsteile der Verbindung des Beispiels 1 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

II 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.-% des Wirkstoffs enthält.

III 30 Gewichtsteile der Verbindung des Beispiels 3 werden mit einer Mischung aus 92 Gewichtsteilen pulverförmigem Kieselsäureregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 4 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gew.-% Wirkstoff enthält.

V 20 Teile der Verbindung des Beispiels 5 werden mit 2 Teilen Calciumsalz der Dodecylbenzolsulfonsäure, 2 Teilen Fettalkohol-polyglykolether, 2 Teilen Natriumsalz eines Phenolsulfonsäure-harnstoff-formaldehyd-kondensates und 68 Teilen eines paraffinischen Mineralöls innig vermischt. Man erhält eine stabile ölige Dispersion.

VI Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

VII 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Ml Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gew.-% des Wirkstoffs enthält.

VIII 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.-% des Wirkstoffs enthält.

IX 20 Gewichtsteile der Verbindung des Beispiels 3 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.-% des Wirkstoffs enthält.

Die erfindungsgemässen Mittel können in diesen Anwendungsformen auch zusammen mit anderen Wirkstoffen vorliegen, z. B. Herbiziden, Insektiziden, Wachstumsregulatoren und Fungiziden oder auch mit Düngemitteln vermischt werden. Beim Vermischen mit Fungiziden erhält man dabei in vielen Fällen eine Vergrösserung des Wirkungsspektrums und eine Leistungssteigerung (Synergismus).

Funzigide, die mit den erfindungsgemässen Verbindungen kombiniert werden können, sind beispielsweise Dithiocarbamate und deren Derivate, wie
Ferridimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis--dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis--dithiocarbamat) und
N,N'-Polypropylen-bis-(thiocarbamoyl)-disulfid.
Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazol-acetat,

2,4-Dichlor-6-(o-chloranilino)-s-triazin,
O,O-Diethyl-phthalimidophosphonthionat,
5-Amino-1-[bis-(dimethylamino)-phosphinyl]-3--phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-[Furyl-(2)]-benzimidazol,
Piperidin-1,4-diyl-bis-1-(2,2,2-trichlor-ethyl)--formamid,
2-[Thiazolyl-(4)]-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-[2-(3,5-Dimethyl-2-oxycycohexyl)-2-hydroxyethyl]-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)--alaninat,
D,L-N-(2,6-Dimethyl-phenyl)-N-(2'-methoxyacetyl)-alaninmethylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-marpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,
Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

Die folgenden Beispiele A und B zeigen die biologische Wirkung der neuen Substanzen. Als Vergleichssubstanz diente das 5-Acetamido-2--methyl-trifluormethansulfonsäureanilid (Fluoridamid, US-PS 3 639 474) sowie das 5-Acetamido-2,4-dimethyl-trifluormethansulfonsäureanilid (Mefluidid, US-PS 3 894 078).

Beispiel A: Wirkung bei Getreide

Im Gewächshaus wurden die Getreidearten Weizen (Kolibri) und Gerste (Union) in Kunststoffschalen von 11,5 cm Durchmesser in Torfkultursubstrat, welches ausreichend mit Nährstoffen versorgt war, eingesät. Die Wirkstoffe wurden in unterschiedlichen Aufwandmengen über das Blatt zugeführt. Die Applikation erfolgte bei einer Wuchshöhe der Pflanzen von 10 cm in üblicher Weise durch Spritzen.

Während der Wachstumszeit von 18 Tagen zeigten die behandelten Pflanzen gegenüber der unbehandelten Kontrolle ein deutlich geringeres Längenwachstum, was durch die abschliessenden Längenmessungen bestätigt werden konnte. Hierbei wurden von jeder Behandlungsreihe 100 Pflanzen gemessen.

Ergebnisse:

Sommerweizen (Kolibri)

| Wirkstoff des Beispiels | Aufwandmenge kg/ha | Pflanzenhöhe cm | Pflanzenhöhe relativ |
|---|---|---|---|
| — | — | 26,8 | 100 |
| Fluoridamid | 1,5 | 17,0 | 63,4 |
| | 6,0 | 15,0 | 56,0 |
| 1 | 1,5 | 13,0 | 48,5 |
| | 6,0 | 12,6 | 46,6 |

Sommergerste (Union)

a)

| Wirkstoff des Beispiels | Aufwandmenge kg/ha | Pflanzenhöhe cm | Pflanzenhöhe relativ |
|---|---|---|---|
| — | — | 26,0 | 100 |
| Fluoridamid | 1,5 | 20,0 | 76,9 |
| | 6,0 | 16,0 | 61,5 |
| 1 | 1,5 | 13,0 | 50,0 |
| | 6,0 | 13,0 | 50,0 |

b)

| Wirkstoff des Beispiels | Aufwandmenge kg/ha | Pflanzenhöhe cm | relativ |
|---|---|---|---|
| — | — | 28,0 | 100 |
| Mefluidide | 0,25 | 27,0 | 96,4 |
| | 0,75 | 18,0 | 64,3 |
| | 1,50 | 17,5 | 62,5 |
| | 3,00 | 16,0 | 57,1 |
| 66 | 0,25 | 26,5 | 94,6 |
| | 0,75 | 16,0 | 57,1 |
| | 1,50 | 16,0 | 57,1 |
| | 3,00 | 15,0 | 53,6 |

Beispiel B: Wirkung auf Gras bzw. Rasen

In einem Vegetationsversuch in Mitscherlichgefässen wurde auf einem lehmigen Sandboden Rasen der folgenden Standard-Mischung angesät: Agrostis tenuis 10%, Cynosurus cristatus 10%, Festuca rubra 15%, Lolium perenne 35% und Poa pratensis 30%. Die Düngung wurde mit 1,5 g N als Ammonnitrat und 1 g $P_2O_5$ als sec. Kaliumphosphat vorgenommen. Nach 2 vorangegangenen Schnitten wurden die Wirkstoffe in verschiedenen Aufwandmengen bei einer Wuchshöhe von 4 cm in üblicher Weise gespritzt. 20 Tage nach der Behandlung wurde die Wuchshöhe bestimmt; nach erfolgtem 1. Schnitt wurde der Wiederaufwuchs über 4 Wochen bis hin zum 2. Schnitt beobachtet und ebenfalls Längenmessungen durchgeführt, um die Nachwirkung zu überprüfen. Gegenüber der Kontrolle reagierten die Pflanzen aus den Behandlungsreihen mit sehr starken Kürzungen. Die Nachwirkung ist besser zu beurteilen als bei der zum Vergleich herangezogenen bekannten Substanz, wie nachstehende Tabelle zeigt.

| Wirkstoff des Beispiels | Aufwandmenge kg/ha | 1. Schnitt Pflanzenhöhe cm | relativ | 2. Schnitt Pflanzenhöhe cm | relativ |
|---|---|---|---|---|---|
| — | — | 14,3 | 100 | 11,0 | 100 |
| Flouridamid | 0,75 | 13,5 | 94,4 | 11,0 | 100 |
| | 0,75 | 12,5 | 87,4 | 10,0 | 90,9 |
| 1 | 0,5 | 5,5 | 38,5 | 8,0 | 72,7 |
| | 0,75 | 4,5 | 31,5 | 7,0 | 63,6 |

**Patentansprüche**

1. Substituierte Sulfonsäureanilide der Formel (I)

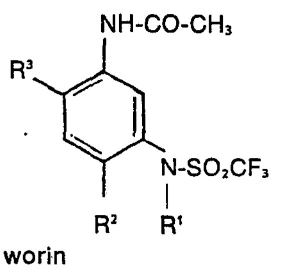

(I)

worin

$R^1$ einen gegebenenfalls durch Halogen, Cyano, Thiocyanato, Azido, Nitro, einen $C_{1-4}$-Alkoxy-, $C_{6-10}$-Aryloxy-, $C_{1-4}$-Alkylthio-, $C_{6-8}$-Arylthio-, $C_{1-4}$--Alkylsulfinyl-, $C_{1-4}$-Alkylsulfonyl-, Arylsulfinyl-, Arylsulfonyl-, $C_{1-5}$-Alkanoyl-, $C_{1-5}$-Alkoxycarbonyl-, $_{1-5}$-Alkyl- oder -Dialkylcarbamoyl-, $C_{1-4}$-Alkanoylamino-, $C_{1-4}$-Halogenalkanoylamino-, $C_{1-6}$--Alkanoyloxy-, $C_{1-4}$-Alkanoylthio-, $C_{1-4}$-Alkoxycarbonylamino-, $C_{7-13}$-Aroyloxy-, $C_{1-6}$-Alkyl- oder -Dialkylamino-, $C_{6-12}$-Arylamino-, $C_{1-6}$-Alkyl- oder -Dialkylcarbamoylamino-, $C_{1-6}$-Alkyl- oder -Dialkylcarbamoyloxy-, $C_{1-4}$-Alkyl- oder -Dialkylaminosulfonyloxyrest oder Kombinationen dieser Gruppen substituierten Alkyl-, Alkenyl-, Alkinyl-, Cycloalkyl-, Cycloalkenyl- (mit jeweils bis zu 12 Kohlenstoffatomen) oder Arylalkylrest mit 7-14 Kohlenstoffatomen, Cyano, Thiocyanato, einen gegebenenfalls $C_{1-4}$-alkylsubstituierten Amino-, Aminosulfonyl-, Guanyl-, $N^2$-Hydroxyguanyl-, Hydroximoyl- oder $C_{1-5}$-Alkoxyimidoylrest, einen gegebenenfalls halogensubstituierten $C_{1-4}$-Alkylsulfenyl- oder $C_{1-4}$-Alkylsulfinylrest oder einen gegebenenfalls halogen- und/oder $C_{1-4}$-alkylsubstituierten Arylsulfinyl- oder Arylsulfonylrest mit bis zu 10 Kohlenstoffatomen im Arylrest bedeutet,

$R^2$ und $R^3$ gleich oder verschieden sind und Halogen, Cyano, Thiocyanato, Nitro, einen gegebenenfalls halogensubstituierten $C_{1-4}$-Alkyl-, $C_{1-4}$-Alkoxy-, $C_{1-4}$-Alkylthio-, $C_{1-4}$-Alkylsulfinyl-, $C_{1-4}$-Alkylsulfonyl- oder $C_{1-4}$-Alkanoylrest bedeuten.

2. Verfahren zur Herstellung der substituierten Sulfonsäureanilide der allgemeinen Formel (I) gemäss Anspruch 1, dadurch gekennzeichnet, dass man

a) Verbindungen der Formel (II)

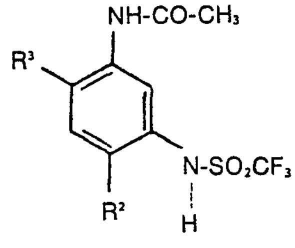

(II)

worin $R^2$ und $R^3$ die oben genannten Bedeutungen besitzen, mit einer Verbindung der allgemeinen Formel (III)

$R^1$-L (III)

worin $R^1$ die oben genannten Bedeutungen besitzt und L eine nucleophil verdrängbare Austrittsgruppe, insbesondere Halogen, Hydrogensulfat, Halogensulfonat, einen gegebenenfalls substituierten Alkylsulfonyloxy-, Arylsulfonyloxy-, Alkylsulfat-, Oxonium-, Sulfonium- oder Ammoniumrest bedeutet, gegebenenfalls in Gegenwart eines Verdünnungsmittels und/oder eines Säurefängers und/oder eines Reaktionsbeschleunigers bei Temperaturen zwischen 0 und 170°C umsetzt, oder

b) Verbindungen der Formel (IV)

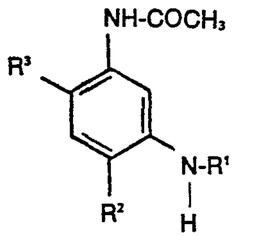

(IV)

worin $R^1$, $R^2$ und $R^3$ die oben genannten Bedeutungen haben, mit einem Sulfonierungsmittel der allgemeinen Formel (V)

$CF_3$-$SO_2$-Z (V)

worin Z eine Austrittsgruppe, insbesondere Halogen oder den Anhydridrest $CF_3$-$SO_2O$- bedeutet, gegebenenfalls in Gegenwart eines Verdünnungsmittels und gegebenenfalls in Gegenwart einer organischen oder anorganischen Base, bei Temperaturen zwischen −60 und +120°C umsetzt.

3. Pflanzenwachstumsregulierendes Mittel enthaltend mindestens eine Verbindung gemäss Anspruch 1.

4. Pflanzenwachstumsregulierendes Mittel enthaltend mindestens eine Verbindung gemäss Anspruch 1 und einen flüssigen oder festen Trägerstoff.

5. Verfahren zur Regulierung des Pflanzenwachstums, dadurch gekennzeichnet, dass man mindestens eine Verbindung gemäss Anspruch 1 auf die Pflanzen oder deren Lebensraum einwirken lässt.

6. Verfahren zur Herstellung von das Pflanzenwachstum regulierenden Mitteln, dadurch gekennzeichnet, dass man mindestens eine Verbindung gemäss Anspruch 1 mit festen oder flüssigen Trägerstoffen mischt.

7. Verwendung von Verbindungen gemäss Anspruch 1 als Wachstumsregulatoren.

8. Verbindung, ausgewählt aus der Gruppe, bestehend aus:
5-Acetamido-N'-cyano-2,4-dimethyl-trifluormethansulfonsäureanilid,
5-Acetamido-N'-cyano-2-methyl-trifluormethansulfonsäureanilid,
5-Acetamido-2,4-dimethyl-N'-methoxy-trifluormethansulfonsäureanilid,
5-Acetamido-2,4-dimethyl-N'-propargyl-trifluormethansulfonsäureanilid,
5-Acetamido-N'-acetyloxymethyl-2,4-dimethyl-trifluormethansulfonsäureanilid,
5-Acetamido-N'-propionyloxymethyl-2,4-dimethyl-trifluormethansulfonsäureanilid,
5-Acetamido-N'-(4'-chlorphenyl)-methyl-2,4-dimethyl-trifluormethansulfonsäureanilid.

9. Pflanzenwachstumsregulierendes Mittel, enthaltend mindestens eine Verbindung ausgewählt aus der Gruppe gemäss Anspruch 8.

**Claims**

1. A substituted sulfonic acid anilide of the formula (I)

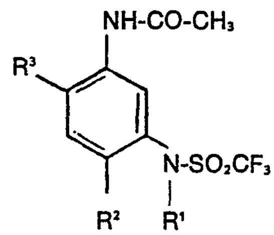

(I)

where

$R^1$ is alkyl, alkenyl, alkynyl, cycloalkyl or cycloalkenyl (each of up to 12 carbon atoms) or arylalkyl (of 7 to 14 carbon atoms), each of which may be unsubstituted or substituted by halogen, cyano, thiocyanato, azido, nitro, $C_{1-4}$-alkoxy, $C_{6-10}$-aryloxy, $C_{1-4}$-alkylthio, $C_{6-8}$-arylthio, $C_{1-4}$-alkylsulfinyl, $C_{1-4}$-alkylsulfonyl, arylsulfinyl, arylsulfonyl, $C_{1-5}$-alkanoyl, $C_{1-5}$-alkoxycarbonyl, $C_{1-5}$-alkylcarbamyl, di-($C_{1-5}$-alkyl)-carbamyl, $C_{1-4}$-alkanoylamino, $C_{1-4}$-haloalkanoylamino, $C_{1-6}$-alkanoyloxy, $C_{1-4}$-alkanoylthio, $C_{1-4}$-alkoxycarbonylamino, $C_{7-13}$-aroyloxy, $C_{1-6}$-alkylamino, di-($C_{1-6}$-alkyl)-amino, $C_{6-12}$-arylamino, $C_{1-6}$-alkylcarbamylamino, di-($C_{1-6}$-alkyl)-carbamylamino, $C_{1-6}$-alkylcarbamyloxy, di-($C_{1-6}$-alkyl)-carbamyloxy, $C_{1-4}$-alkylaminosulfonyloxy or di-($C_{1-4}$-alkyl)-aminosulfonyloxy or combinations of these groups, or is cyano, thiocyanato, unsubstituted or $C_{1-4}$-alkyl-substituted amino, aminosulfonyl, guanyl, $N_2$-hydroxyguanyl, hydroximoyl or $C_{1-5}$-alkoxyimidoyl, or is unsubstituted or halogen-substituted $C_{1-4}$-alkylsulfenyl or $C_{1-4}$-alkylsulfinyl, or is unsubstituted or halogen- and/or $C_{1-4}$-alkyl-substituted arylsulfinyl or arylsulfonyl, where aryl is of up to 10 carbon atoms,

$R^2$ and $R^3$ are identical or different and each is halogen, cyano, thiocyanato, nitro or unsubstituted or halogen-substituted $C_{1-4}$-alkyl, $C_{1-4}$-alkoxy, $C_{1-4}$-alkylthio, $C_{1-4}$-alkylsulfinyl, $C_{1-4}$-alkylsulfonyl or $C_{1-4}$-alkanoyl.

2. A process for the preparation of a substituted sulfonic acid anilide of the general formula (I) as claimed in claim 1, characterized in that

a) a compound of the formula (II)

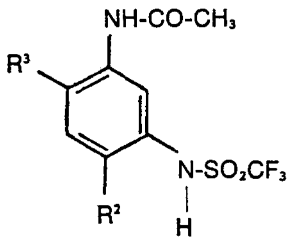

(II)

where $R^2$ and $R^3$ have the above meanings, is reacted with a compound of the general formula (III)

$R^1$-L (III)

where $R^1$ has the above meanings and L is a nucleophilically displaceable leaving group, especially halogen, bisulfate or halosulfonate, or unsubstituted or substituted alkylsulfonyloxy, arylsulfonyloxy, alkylsulfate, oxonium, sulfonium or ammonium, in the presence or absence of a diluent and/or of an acid acceptor and/or of a reaction accelerator at from 0° to 170°C, or

b) a compound of the formula (IV)

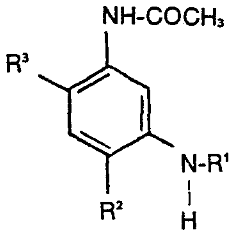

(IV)

where $R^1$, $R^2$ and $R^3$ have the above meanings, is reacted with a sulfonating agent of the general formula (V)

$CF_3$-$SO_2$-Z (V)

where Z is a leaving group, especially halogen or an anhydride radical $CF_3$-$SO_2O$-, in the presence or absence of a diluent and in the presence or absence of an organic or inorganic base, at from −60° to +120°C.

3. A plant-growth-regulating agent containing at least one compound as claimed in claim 1.

4. A plant-growth-regulating agent containing at least one compound as claimed in claim 1 and a liquid or solid carrier.

5. A process for regulating plant growth, characterized in that at least one compound as claimed in claim 1 is allowed to act on the plants or their habitat.

6. A process for the production of plant-growth-regulating agents, characterized in that at least one compound as claimed in claim 1 is mixed with a solid or liquid carrier.

7. Use of a compound as claimed in claim 1 as a growth-regulating agent.

8. A compound selected from the group comprising trifluoromethanesulfonic acid 5-acetamido-$N^1$-cyano-2,4-dimethyl-anilide, trifluoromethanesulfonic acid 5-acetamido-$N^1$-cyano-2-methyl-anilide, trifluoromethanesulfonic acid 5-acetamido-2,4-dimethyl-$N^1$-methoxymethyl-anilide, trifluoromethanesulfonic acid 5-acetamido-2,4-dimethyl-$N^1$-propargyl-anilide, trifluoromethanesulfonic acid 5-acetamido-$N^1$-acetoxymethyl-2,4-dimethyl-anilide, trifluoromethanesulfonic acid 5-acetamido-$N^1$-propionyloxymethyl-2,4-dimethyl-anilide and trifluoromethanesulfonic acid 5-acetamido-$N^1$-(4'-chlorophenyl)-methyl-2,4-dimethyl-anilide.

9. A plant-growth-regulating agent containing at least one compound selected from the group as claimed in claim 8.

**Revendications**

1. Anilides d'acide sulfonique substitués, de formule (I)

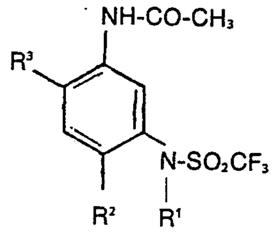

(I)

dans laquelle

$R^1$ représente un reste alkyle, alcényle, alcinyle, cycloalkyle, cycloalcényle (chacun ayant jusqu'à 12 atomes en carbone) ou un reste arylalkyle à 7-14 atomes de carbone, éventuellement substitués par halogène, cyano, thiocyanato, azido, nitro, un reste $C_{1-4}$-alcoxy, $C_{6-10}$-aryloxy, $C_{1-4}$-alkylthio, $C_{6-8}$-arylthio, $C_{1-4}$-alkylsulfinyle, $C_{1-4}$-alkylsulfonyle, arylsulfinyle, arylsulfonyle, $C_{1-5}$-alcanoyle, $C_{1-5}$-alcoxy-carbonyle, $C_{1-5}$-alkyle ou -dialkylcarbamoyle, $C_{1-4}$-alcanoylamino, $C_{1-4}$-halogenalcanoylamino, $C_{1-6}$-alcanoyloxy, $C_{1-4}$-alcanoylthio, $C_{1-4}$-alcoxycarbonylamino, $C_{7-13}$-aroyloxy, $C_{1-6}$-alkyl- ou dialkylamino, $C_{6-12}$-arylamino, $C_{1-6}$-alkyl- ou dialkylcarbamoylamino, $C_{1-6}$-alkyl- ou dialkylcarbamoyloxy, $C_{1-4}$-alkyl- ou dialkylaminosulfonyloxy ou des combinaisons de ces groupes, cyano, thiocyanato, un reste amino, aminosulfonyle, guanyle, $N^2$-hydroxyguanyle, hydroximoyle ou $C_{1-5}$-alcoxyimidoyle éventuellement substitué par $C_{1-4}$-alkyle, un reste $C_{1-4}$-alkylsulfényle ou $C_{1-4}$-alkylsulfinyle éventuellement substitué par halogène, ou un reste arylsulfinyle ou arylsulfonyle, ayant jusqu'à 10 atomes de carbone dans le reste aryle et éventuellement substitué par halogène et/ou $C_{1-4}$-alkyle,

$R^2$ et $R^3$ sont identiques ou différents et représentent halogène, cyano, thiocyanato, nitro, un reste $C_{1-4}$-alkyle, $C_{1-4}$-alcoxy, $C_{1-4}$-alkylthio, $C_{1-4}$-alkylsulfinyle, $C_{1-4}$-alkylsulfonyle ou $C_{1-4}$-alcanoyle, éventuellement substitué par halogène.

2. Procédé de préparation d'anilides d'acide sulfonique substitués de formule générale (I) selon la revendication 1, caractérisé par le fait que:

a) on fait réagir, à des températures comprises entre 0 et 170°C, des composés de formule (II)

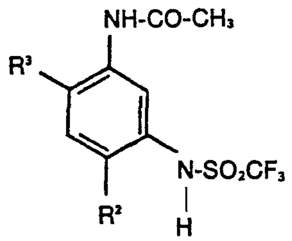

(II)

dans laquelle $R^2$ et $R^3$ ont les significations indiquées ci-dessus, avec un composé de formule générale (III)

$R^1$-L (III)

dans laquelle $R^1$ a les significations sus-indiqués et L représente un groupe déplaçable, nucléophile, en particulier halogène, sulfate acide, halogéno-sulfonate, un reste alkylsulfonyloxy, arylsulfonyloxy, alkylsulfate, oxonium, sulfonium ou ammonium éventuellement substitué, en présence éventuellement d'un diluant et/ou d'un fixateur d'acide et/ou d'un accélérateur de réaction ou,

b) on fait réagir, à des températures comprises entre −60 et +130°C, des composés de formule (IV)

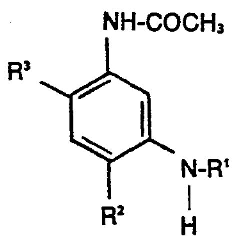

(IV)

dans laquelle $R^1$, $R^2$ et $R^3$ ont les significations susindiquées, avec un agent de sulfonation de formule générale (V)

$CF_3$-$SO_2$-Z (V)

dans laquelle Z représente un groupe de sortie, en particulier halogène ou le reste anhydride $CF_3$-$SO_2$-O-, éventuellement en présence d'un diluant et, éventuellement, en présence d'une base organique ou inorganique.

3. Agent de régularisation de la croissance des plantes contenant au moins un composé selon la revendication 1.

4. Agent de régularisation de la croissance des plantes contenant au moins un composé selon la revendication 1 et un support liquide ou solide.

5. Procédé de régularisation de la croissance des plantes, caractérisé par le fait que l'on fait réagir un composé selon la revendication 1 sur les plantes ou leur biotope.

6. Procédé de préparation d'agent de régularisation de la croissance des plantes, caractérisé par le fait qu'on mélange ou moins un composé selon la revendication 1, avec des supports solides ou liquides.

7. Emploi de composés selon la revendication 1, comme régulateurs de croissance.

8. Composé, choisi dans le groupe constitué d'anilides des acides suivants:
5-acétamido-N'-cyano-2,4-diméthyl-trifluorméthansulfonique,
5-acétamido-N'-cyano-2-méthyl-trifluorméthansulfonique,
5-acétamido-2,4-diméthyl-N'-méthoxyméthyl-trifluormethansulfonique,
5-acétamido-2,4-diméthyl-N'-propargyl-trifluorméthansulfonique,
5-acétamido-N'-acétyloxyméthyl-2,4-diméthyl-trifluormethansulfonique,
5-acétamido-N'-propionyloxyméthyl-2,4-diméthyl--trifluormethansulfonique,
5-acétamido-N'-(4'-chlorphényl)-méthyl-2,4-diméthyl-trifluormethansulfonique.

9. Agent de régularisation de la croissance des plantes contenant au moins un composé choisi dans le groupe selon la revendication 8.